(19) Europäisches Patentamt
European Patent Office
Office européen des brevets

(11) Numéro de publication: **0 135 402**
**B1**

(12) # FASCICULE DE BREVET EUROPEEN

(45) Date de publication du fascicule du brevet: **28.10.87**

(51) Int. Cl.⁴: **C 07 D 221/28**, A 61 K 31/435

(21) Numéro de dépôt: **84401291.4**

(22) Date de dépôt: **21.06.84**

(54) **Antagonistes périphériques des opiacés, procédé d'obtention et compositions pharmaceutiques les contenant.**

(30) Priorité: **24.06.83 FR 8310526**

(43) Date de publication de la demande:
**27.03.85 Bulletin 85/13**

(45) Mention de la délivrance du brevet:
**28.10.87 Bulletin 87/44**

(84) Etats contractants désignés:
**AT BE CH DE FR GB IT LI LU NL SE**

(56) Documents cités:
**FR - A - 1 179 915**
**US - A - 4 176 186**

**CHEMICAL ABSTRACTS REGISTRY HANDBOOK, "NUMBER SECTION - 1977 SUPPLEMENT", page 1827RF, no. 63868-46-2, Columbus, Ohio, US;**
**HELVETICA CHIMICA ACTA, vol. 39, no. 49, fasciculus II, 1956, pages 429-440, Bâle, CH; J. HELLERBACH et al.: "Hydroxy-morphinane. (-)-3-Hydroxy-N-allyl-morphinan und verwandte Verbindungen"**
**JOURNAL OF MEDICINAL CHEMISTRY, vol. 25, novembre 1982, pages 1278-1280, American Chemical Society, Columbus, Ohio, US; R.J. KOBYLECKI et al.: "N-Methylnalorphine: *Definition of N-allyl conformation for antagonism at the opiate receptor*"**
**CHEMICAL ABSTRACTS, vol. 100, no. 3, 16 janvier 1984,**

(56) Entgegenhaltungen: (Fortsetzung)
**abrégé no. 17504y, Columbus, Ohio, US; A. NOTARNICOLA et al.: "Relative ability of *N-methylnalorphine and N-methyllevallorphan to prevent antinociception and intestinal transit inhibition in morphine treated rats*"**

**Le dossier contient des informations techniques présentées postérieurement au dépôt de la demande et ne figurant pas dans le présent fascicule.**

(73) Titulaire: **SANOFI, société anonyme, 40, Avenue George V, F-75008 Paris (FR)**
(84) Etats contractants désignés: **BE CH DE FR GB LI LU NL SE AT**

(73) Titulaire: **MIDY S.p.A., Société Anonyme dite:, Via Piranesi 38, I-20137 Milano (IT)**
(84) Etats contractants désignés: **IT**

(72) Inventeur: **Bianchetti, Alberto, Via Corridoni 11, I-20122 Milano (IT)**
Inventeur: **Nisato, Dino, Viale Golgi 80, Pavia (IT)**
Inventeur: **Manara, Luciano, Via Novella 2, I-14040 Pietra Marazzi (Allessandria) (IT)**
Inventeur: **Sacilotto, Roberto, Via Sirio 3/L, I-20060 Cassina de Pecchi Milano (IT)**

(74) Mandataire: **Gillard, Marie-Louise et al, Cabinet Beau de Loménie 55, Rue d'Amsterdam, F-75008 Paris (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

EP 0 135 402 B1

ACTORUM AG

## Description

La présente invention concerne des antagonistes périphériques des opiacés.

Plus particulièrement, la présente invention se réfère à des sels quaternaires dérivés du (−)-3-hydroxy-N-allyl-morphinan ici désigné par sa Dénomination Commune Internationale «lévallorphane».

Malgré les divers problèmes liès à l'usage des opiacés, tels que la tolérance et la dépendance physique, les médicaments morphiniques restent sans égaux dans le traitement de la douleur violente.

Un effet secondaire, souvent sérieux, des morphiniques est représenté par la constipation qui est dûe à leur action locale au niveau des récepteurs intestinaux des opiacés.

Pour pallier ce type d'effet secondaire très gênant pour le patient, il a été proposé qu'un antagoniste des narcotiques ayant une faible capacité de pénétration à travers la barrière hématoméningée pourrait réduire d'une façon significative la constipation provoquée par les médicaments morphiniques sans en réduire substantiellement les effets analgésiques.

Le brevet US 4 176 186 décrit des dérivés quaternaires d'antagonistes morphiniques qui préviennent ou éliminent les effets secondaires des opiacés sur la motilité intestinale. Parmi les composés revendiqués par le brevet ci-dessus, les composés préférés sont les dérivés quaternaires de la N-allylnoroxymorphone, ci-après désignée «naloxone», dont le bromométhylate est particulièrement préféré.

Un autre composé, le bromoallylate de lévallorphane, a été décrit comme antagoniste périphérique des opiacés (Life Sci. 1982, 31, 2261–2264).

Selon la demande de brevet européen EP-A-0 113 632 en faisant réagir le lévallorphane avec un halogénure de méthyle, on obtient un composé, désigné «halométhylate de lévallorphane», de formule

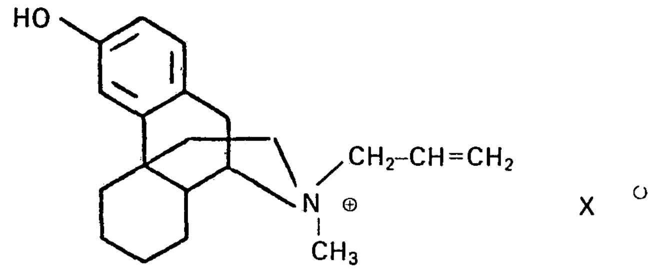

I

dans laquelle X représente le chlore, le brome ou l'iode, qui possède une capacité de pénétration, de la barrière hématoméningée pratiquement nulle tout en conservant une très bonne activité antagoniste périphérique.

Le terme «halométhylate de lévallorphane» est utilisé dans la demande de brevet français ci-dessus pour distinguer le produit préparé comme indiqué ci-dessus de celui obtenu par réaction d'un halogénure d'allyle sur le (−)-3-hydroxy-N-méthylmorphinane, dont la Dénomination Commune Internationale est «lévorphanol», qui possède également la formule I ci-dessus, mais des caractéristiques physicochimiques différentes et qui est inactif sur le plan de l'activité antagoniste, périphérique ou centrale.

En effet, la quaternarisation de l'atome d'azote en position 17 introduit un nouveau centre d'asymétrie et les deux diastéreoisomères peuvent être obtenus par synthèse differenciée comme indiqué ci-dessus et dans J. Med. Chem. 1982, 25, 1278–1280 où est décrite la préparation des deux diastéreoisomères dérivés de la nalorphine et de la morphine.

Les halométhylates de lévallorphane décrits dans la demande de brevet français ci-dessus posent cependant certains problèmes dûs aux caractéristiques particulières des produits; par exemple l'utilisation d'un composé ayant un ion $I^-$ dans un médicament nécessite la surveillance de la fonction thyroïdienne des patients. De leur côté, les composés ayant les ions $Cl^-$ et $Br^-$ requièrent, pour leur préparation, l'utilisation du bromure et du chlorure de méthyle qui sont des produits gazeux, donc pas si facilement maniables.

On a maintenant trouvé qu'en faisant réagir un halométhylate de lévallorphane avec un excès d'un acide pharmaceutiquement acceptable on obtient l'échange de l'ion halogène avec l'anion de l'acide employé et la formation de nouveaux sels de (−)-3-hydroxy-N-méthyl-N-allylmorphinanium ayant la même configuration que l'halométhylate de lévallorphane de départ et qui sont désignés ci-après «sels de méthyllévallorphanium».

On a également trouvé que les nouveaux sels ainsi obtenus possèdent, eux aussi, une très bonne activité antagoniste périphérique des opiacés et une capacité de pénétration de la barrière hématoméningée pratiquement nulle.

Ainsi, la présente invention a pour objet, selon un des ses aspects, des sels de méthyllévallorphanium de formule

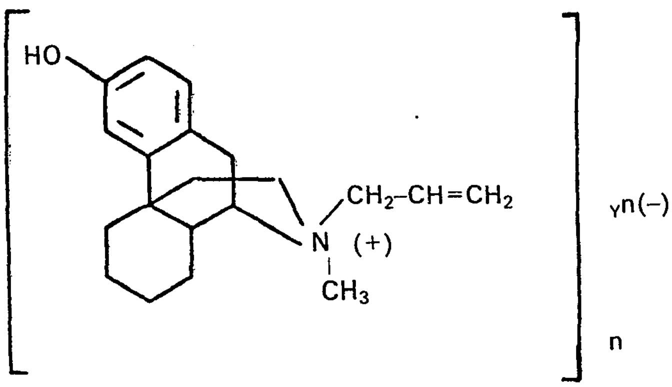

II

dans laquelle $Y^{n(-)}$ représente l'anion d'un acide pharmaceutiquement acceptable autre qu'un anion halogène et n représente le nombre des charges négatives dudit anion.

L'anion d'acide pharmaceutiquement acceptable dérive de n'importe quel acide couramment utilisé pour salifier les composés destinés à l'usage pharmaceutique et notamment les acides salifiant les opiacés.

Les acides méthanesulfonique, éthanesulfonique, p-toluènesulfonique, 2-napthalènesulfonique, 1,5-naphtalènedisulfonique, sulfurique, sulfurique monométhylester, phosphorique, ascorbique, malonique, maléique, tartrique, fumarique, malique, phytique, citrique, succinique, 4,4'-méthylène-bis- (3-hydroxy-2-naphtoïque) sont des acides salifiant avantageux.

Les anions méthanesulfonate ($CH_3SO_3^-$), ci-après désigné «mésylate», p-toluènesulfonate ($4\text{-}CH_3\text{–}CH_6H_4SO_3^-$), ci-après désigné «tosilate», 2-naphthalènesulfonate ($2\text{–}C_{10}H_7\ SO_3^-$), ci-après désigné «napsilate», maléate acide (cis-HOOC–CH=CH–COO$^-$), sulfate neutre ($SO^2{}_4-$), ci-après désigné simplement «sulfate», et méthylsulfate ($CH_3O\text{–}SO_3^-$) sont particulièrement préférés.

Les sels de méthyllévallorphanium de la présente invention sont préparés, selon un autre aspect de la présente invention, par échange de l'anion halogène d'un halométhylate de lévallorphane, l'iodométhylate de préférence, avec l'anion $Y^{n(-)}$ d'un acide pharmaceutiquement acceptable, par exemple à l'aide d'une résine échangeuse d'ions.

On utilise de préférence une résine de la série AMBERLITE, notamment l'AMBERLITE IRA-400 portant l'anion chlorure. On déplace l'ion chlorure en faisant passer une solution d'un hydroxyde alcalin dans une colonne contenant la résine et on lave à fond la colonne à l'eau distillée jusqu'à pH neutre. On fait ensuite passer dans la colonne contenant la résine neutre une solution aqueuse à 10% de l'acide pharmaceutiquement acceptable choisi et on lave encore à l'eau distillée jusqu'à pH neutre pour obtenir la résine portant l'anion de l'acide employé.

On fait passer une solution aqueuse d'halométhylate de lévallorphane dans la colonne ainsi préparée et on élue avec de l'eau.

Le sel de méthyllévallorphanium ainsi obtenu est isolé par évaporation à sec de la solution aqueuse obtenue et cristallisation dans un solvant approprié.

L'antagonisme à l'action périphérique des opiacés est évalué sur la base de l'inhibition pour cent de l'effet analgésique et de l'effet constipant de la morphine. Le rapport entre la dose qui inhibe de 50% l'effet analgésique de la morphine et la dose qui en inhibe de 50% l'effet constipant représente l'indice de l'activité périphérique.

L'antagonisme à l'effet analgésique de la morphine à été évalué par la méthode de la plaque chaude selon P.A. Janssen et al. (J. Pharm. Pharmacol. 1957, 9, 381–400) en utilisant des souris femelles de 20–22 g, à jeun depuis 18 heures. La morphine a été injectée par voie sous-cutanée à la dose de 24 mg/kg 30 minutes avant le début du test. L'antagoniste a été injecté par voie sous-cutanée 5 minutes avant la morphine. L'activité antagoniste a été exprimée comme DI50 de l'effet antinociceptif de la morphine, calculée sur la base de la régression log dose-temps de réaction des animaux traités avec de différentes doses des produits sous examen.

L'antagonisme à l'effet constipant de la morphine des sels de méthyllévallorphanium de la présente invention a été évalué selon le test décrit par A.F. Green (Br. J. Pharmacol. Chemother. 1959, 14, 26–34), légèrement modifié.

On a utilisé trois groupes de souris femelles de 20 g environ à jeun depuis 20 heures. A un groupe témoin, on a administré par voie orale un repas constitué par 0,2 ml d'un mélange de gomme arabique 5% (6 ml), farine (2 g), et charbon (1 g). Au deuxième groupe on a administré par la voie sous-cutanée 12 mg/kg de morphine et, immédiatement après, le repas au charbon ci-dessus. Le troisième groupe a été traité par l'antagoniste sous examen par voie sous-cutanée, puis, après 5 minutes, par 12 mg/kg de morphine par voie sous-cutanée et, tout-de-suite après, par le repas au charbon. Après trente minutes les animaux ont été sacrifiés pour l'évaluation de la portion intestinale parcourue par le charbon, exprimée en pour cent de la longueur totale de l'intestin grêle.

Les doses qui inhibent de 50% l'effet analgésique (DI50a) et l'effet constipant (DI50c) ont été extrapolées de la droite de régression log dose-réponse en appliquant l'analyse de la variance par régression linéaire.

Les résultats obtenus dans les tests ci-dessus pour deux composés représentatifs de la présente invention, le méthylsulfate de méthyllévallorphanium et le mésylate de méthyllévallorphanium et de quatre produits de référence, à savoir le bromoallylate de lévallorphane, décrit dans Life Sci. 1982, 31, 2261–2264, le sulfométhylate de naloxone, décrit dans le brevet US 4 176 186, le tartrate de lévallorphane et la naloxone, sont consignés dans le tableau I.

Tableau I

| Composé | Antagonisme à l'effet analgésique DI50a mg/kg | Antagonisme à l'effet constipant DI50c mg/kg | DI50a / DI50c |
|---|---|---|---|
| méthylsulfate de méthyllévallorphanium | ~60° | 4,9 (2,9–8,6) | ~12 |
| mésylate de méthyllévallorphanium | >60 | 7,4 (4,4–12,6) | > 8 |
| bromoallylate de lévallorphane | 15,2 (9,7–24) | 15,65 (8,46–28,9) | 0,97 |
| sulfométhylate de naloxone | 1,6 (0,7–3,6) | 5,8 (2,9–11,5) | 0,27 |
| tartrate de lévallorphane | 0,03 (0,01–0,08) | 5,5 (1,7–17,8) | 0.01 |
| naloxone | 0,06 (0,02–0,15) | 0,75 (0,46–1,23) | 0,08 |

° DI50 non déterminable (à 30 mg/kg: inhib. 41%, à 60 mg/kg: inhib. 49%)

De ce tableau il ressort que les composés représentatifs de la présente invention antagonisent l'effet constipant de la morphine avec une DI50c de 4,9 et de 7,4 mg/kg alors qu'un d'entre eux n'antagonise l'effet analgésique de la morphine qu'aux doses maximales injectables et l'autre ne l'antagonise même pas à la dose maximale injectable. Par contre, le dérivé quaternaire de la naloxone décrit dans le brevet US 4 176 186 n'a pas de sélectivité périphérique satisfaisante. En effet, il est plus actif comme antagoniste de l'effet analgésique de la morphine que de l'effet constipant.

De son côté, le bromoallylate de lévallorphane est un bon antagoniste périphérique des opiacés, mais le rapport entre la DI50a et la DI50c est de loin inférieur à celui des composés de la présente invention.

La naloxone et le tartrate de lévallorphane sont bien plus actifs comme antagonistes de l'effet analgésique que comme antagonistes de l'effet constipant.

L'action antagoniste périphérique sélective montrée par les sels de lévallorphanium de la présente invention dans les tests indiqués ci-dessus, associée à une faible toxicité, les rend utiles comme médicaments.

Ainsi, la présente invention a pour objet, selon un aspect ultérieur, des compositions pharmaceutiques à action antagoniste périphérique des opiacés renfermant, en tant qu'ingrédient actif, un sel de méthyllévallorphanium de formule II ci-dessus en mélange avec un excipient pharmaceutique.

Les compositions pharmaceutiques à action antagoniste périphérique des opiacés de la présente invention sont utiles dans le traitement des conditions pathologiques où il existe des taux altérés d'opiacés exogènes ou endogènes, ou une hypersensibilité aux opiacés hors du système nerveux central des mammifères.

Ainsi, les compositions de la présente invention peuvent être administrées aux mammifères, animaux et êtres humains, avec les opiacés afin d'en prévenir les effets secondaires, notamment la constipation, qui dérivent notamment de l'activation des récepteurs situés à la périphérie, sans compromettre l'analgésie, ou n'importe quelle autre action des opiacés, provoquée par stimulation des récepteurs centraux de la part de l'opiacé.

Afin d'obtenir l'effet antagoniste périphérique désiré, la dose de principe actif peut varier entre 0,05 et 200 mg par kg de poids du corps et par jour.

Chaque dose unitaire peut contenir de 1 à 500 mg d'ingrédient actif en combinaison avec un support pharmaceutique. Cette dose unitaire peut être administrée aux mammifères 1 à 4 fois par jour.

Les compositions pharmaceutiques de la présente invention à action antagoniste phériphérique des opiacés peuvent être formulées pour l'administration orale, sublinguale, nasale, sous-cutanée, intramusculaire, intraveineuse, transdermi-

que ou rectale en mélangeant l'ingrédient actif de formule II ci-dessus avec des supports pharmaceutiques classiques.

Les formes unitaires d'administration comprennent les comprimés, les gélules, les poudres, les granules et les solutions ou suspensions orales, les suppositoires ainsi que les ampoules utiles pour une administration parentérale.

Lorsqu'on prépare une composition solide sous forme de comprimés, on mélange l'ingrédient actif principal avec un véhicule, pharmaceutique, tel que la gélatine, l'amidon, le lactose, le stéarate de magnésium, le talc, la gomme arabique ou analogues. On peut enrober les comprimés de saccharose ou d'autres matières appropriées ou encore on peut les traiter d'une autre manière de telle sorte qu'ils aient un activité prolongée ou retardée et qu'ils libèrent continuellement une quantité prédéterminée de principe actif.

On obtient une préparation en gélules en mélangeant l'ingrédient actif avec un diluant et en versant le mélange obtenu dans des gélules molles ou dures.

Une préparation sous forme de sirop ou d'elixir peut contenir l'ingrédient actif avec un édulcorant éventuellement hypocalorique, du méthylparaben et du propylparaben comme antiseptiques, ainsi qu'un agent donnant du goût et un colorant approprié.

Les poudres ou les granulats dispersibles dans l'eau peuvent contenir l'ingrédient actif en mélange avec des agents de dispersion ou des agents mouillants, ou des agents de mise en suspension, tels que la polyvinylpyrrolidone de même qu'avec des édulcorants ou des correcteurs du goût.

Pour une application rectale, on recourt à des suppositoires qui sont préparés avec des liants fondant à la température rectale, par exemple du beurre de cacao ou des polyéthylèneglycols.

Pour une administration orale en gouttes ou pour une administration parentérale, on utilise des suspensions aqueuses, des solutions salines isotoniques stériles et injectables qui contiennent des agents de dispersion et/ou des mouillants pharmacologiquement compatibles, par exemple le propylèneglycol ou le butylèneglycol.

Le principe actif peut être formulé également sous forme de microcapsules, éventuellement avec un ou plusieurs supports ou additifs.

Les compositions de la présente invention peuvent contenir, à côté du sel de méthyllévallorphanium, d'autres principes actifs tels que, par exemple, des analgésiques agonistes ou agonistes/antagonistes des opiacés comme la morphine, la codéine, la buprénorphine, des antitussifs ou d'autres médicaments appropriés.

Les exemples suivants illustrent l'invention sans toutefois la limiter.

Preparation

On chauffe 6 heures sous agitation à la température externe de 80 °C un mélange de 5 g de lévallorphane, 70 ml de diméthylformamide et 6,7 g d'iodure de méthyle, puis on concentre sous pression réduite tout en éliminant le solvant et l'excès d'alkylant par addition d'acétone. Par refroidissement de la solution acétonique on obtient un solide qui, après filtration et séchage sous pression réduite, fond à 225–227 °C Par recristallisation dans 50 ml d'éthanol à 90%, on obtient 5 g d'un produit blanc; p>f. 230 à 232 °C. Après deux autres cristallisations jusqu'à l'obtention d'un pouvoir rotatoire constant, on obtient, avec un rendement de 40%, l'iodométhylate de lévallorphane pur; p.f. 232 à 234 °C (méthode de Tottoli); $[\alpha]_D^{20} = -64° \pm 1°$ (méthanol, c=0,2%).

Le spectre protonique RMN effectué à 250 MHz dans du diméthylsulfoxide deutéré, en utilisant le tétraméthylsilane en tant que standard interne, présente un singulet à 3,20 ± 0,02 ppm, relatif au groupe méthyle en position 17.

L'autre diastéreoisomère, iodoallylate de lévallorphanol, présente le même singulet à 3,04 ± 0,02 ppm.

Exemple 1

Dans une colonne en verre ayant un diamètre de 2,5 cm on introduit 100 g de résine échangeuse d'ions AMBERLITE IRA-400 (marque enregistrée, B.D.H.) sous forme de chlorhydrate. Après élution avec 1000 ml d'hydroxyde de sodium à 10%, on lave à l'eau distillée jusqu-à pH neutre, on élue avec une solution à 10% d'acide méthanesulfonique jusqu'à pH acide, puis on lave de nouveau à l'eau jusqu'à pH neutre. Sur la colonne contenant la résine sous forme de mésylate ainsi obtenue on verse une solution de 3 g de iodométhylate de l'évallorphane (décrit dans la PREPARATION) dans de l'eau distillée, puis on élue avec de l'eau. On évapore l'eau à siccité sous pression réduite et on cristallise le résidu dans l'éthanol. On obtient ainsi 2 g de mésylate de méthyllévallorphanium (formule II, $Y^{n(-)} = CH_3SO_3^-$); p.f. 265–267 °C; $[\alpha]_D^{20} = -64,9°$ (c=0,5%, méthanol).

Exemple 2

En opérant comme décrit dans l'exemple 1, par échange de l'ion $I^-$ de l'iodométhylate de lévallorphane (3 g) avec l'ion méthylsulfate sur une colonne AMBERLITE IRA-400 traitée avec de l'acide sulfurique monométhylester, on obtient 1,9 g de méthylsulfate de méthyllévallorphanium (formule II, $Y^{n(-)} = CH_3OSO_3$; p.f. 263–265 °C; $[\alpha]_D^{20} = -63,8°$ (c=0,5%, méthanol).

Exemple 3

Suivant le mode operatoire décrit dans l'exemple 1, à partir de 3 g de iodométhylate de lévallorphane, sur une colonne contenant de la résine AMBERLITE IRA-400 traitée avec de l'acide maléique, on obtient 2,1 g de maléate acide de méthyllévallorphanium (formule II, $Y^{n(-)}$=cis $HOOC-CH=CH-COO^-$) cristallisé dans l'isopropanol, p.f. 209–211 °C; $[\alpha]_D^{20} = -61,2°$ (c=0,5%, méthanol).

Exemples 4 à 7

En opérant comme décrit dans l'exemple 1, à partir de 3 g de iodométhylate de lévallorphane sur une colonne contenant de la résine AMBERLITE IRA-400 traitée respectivement avec les acides fumarique, L(+) tartrique, perchlorique et phosphorique, on obtient les sels de lévallorphanium suivants:

– fumarate acide de méthyllévallorphanium (formule II, $Y^{n(-)}$=trans–HOOC–CH = CH–$COO^-$) p.f. 235 °C (déc); $[\alpha]_D^{20}$ = −63,6° (c=0,5% méthanol); Ex. 4;

– L(+) tartrate acide de méthyllévallorphanium (formule II $Y^{n(-)}$=L(+)–HOOC–CHOH–CHOH–$COO^-$); p.f. 223–225 °C; Ex. 5;

– perchlorate de méthyllévallorphanium (formule II, $Y^{n(-)}$=$C104^-$); p.f. 258–260 °C; $[\alpha]_D^{20}$ = −65,3°(c=0,5% méthanol; Ex. 6;

– phosphate diacide de méthyllévallorphanium-(formule II, $Y^{n(-)}$=$H2PO4^-$); p.f. 235–237 °C; $[\alpha]_D^{20}$ = −62,5° (c=0,5% méthanol), Ex. 7

Exemple 8

On prépare des comprimés à base d'un des composés décrits dans les exemples 1 à 7, ayant la composition suivante:

| | |
|---|---|
| principe actif | 50 mg |
| lactose | 145 mg |
| avicel | 100 mg |
| stéarate de magnésium | 5 mg |

en broyant l'ingredient activ jusqu'à une dimension des particules de 0,4 mm, en le faisant passer à travers un tamis de 0,4 mm d'ouverture de maille, en mélangeant la matière broyée avec les autres constituants et en comprimant pour former les comprimés.

De la même façon, on prépare des comprimés contenant 40 mg de principe actif.

Exemple 9

On prépare des gélules à base d'un des composés décrits dans les exemples 1 à 7, ayant la composition suivante:

| | |
|---|---|
| principe actif | 15 mg |
| lactose | 120 mg |
| stéarate de magnésium | 5 mg |

en mélangeant intimement les ingrédients ci-dessus et en versant le mélange dans des gélules de gélatine dure.

De la même façon, on prépare des gélules contenant 25 mg de principe actif.

Exemple 10

10 000 gélules avec une teneur en substance active de 50 mg sont préparées à partir des constituants suivants: 500 g d'un des composés décrits dans les exemples 1 à 7, 495 g de cellulose microcristalline, 5 g de gel de silice amorphe. Les constituants ci-dessus sont bien mélangés et on les introduit dans des gélules de gélatine dure de dimension 4.

Exemple 11

On prépare une solution aqueuse stérile appropriée pour une utilisation parentérale en ampoules à base d'un des composés décrits dans les exemples 1 à 7, ayant la composition suivante:

| | |
|---|---|
| principe actif | 10 mg |
| eau pour préparation injectable q.s.p. | 2 ml |

Exemple 12

On prépare des suppositoires à base d'un des composés décrits dans les exemples 1 à 7, ayant la composition suivante:

| | |
|---|---|
| principe actif | 50 mg |
| lactose | 250 mg |
| masse pour suppositoires q.s.p. | 1,7 g |

On mélange le principe actif avec le lactose et on met le mélange uniformément en suspension dans la masse pour suppositoires fondue.

On verse la suspension dans des moules refroidis pour former des suppositoires d'un poids de 1,7 g.

Exemple 13

On prépare des comprimés dragéifiés contenant chacun 30 mg d'un des composés décrits dans les exemples 1 à 7 en utilisant comme excipient du talc, du lactose, de l'amidon de maïs, de l'alginate de sodium, du sucre semoulé, du sucre cristallisé, du stéarate de magnésium, de la gomme laque blanche, de la gélatine alimentaire, de l'érythrosine, du bioxyde de titane et de la cire blanche.

**Revendications pour les etats contractants: BE, CH, DE, FR, GB, IT, LI, LU, NL, SE**

1. Sel de méthyllévallorphanium de formule

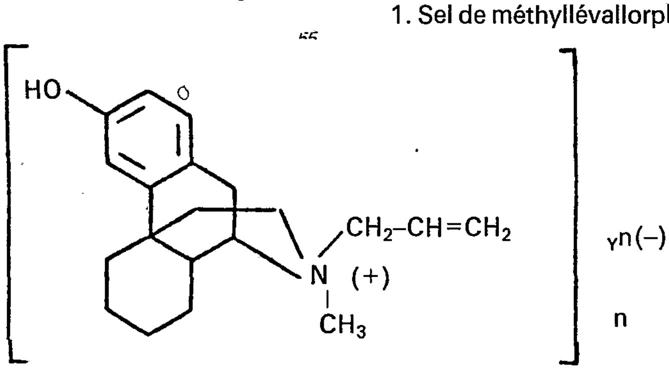

dans laquelle $Y^{n(-)}$ représente l'anion, autre qu'un anion halogène, d'un acide pharmaceutiquement acceptable et n représente le nombre des charges négatives dudit anion.

2. Mésylate de méthyllévallorphanium.

3. Maléate acide de méthyllévallorphanium.

4. Méthylsulfate de méthyllévallorphanium.

5. Procédé pour la préparation de sels de méthyllévallorphanium de formule

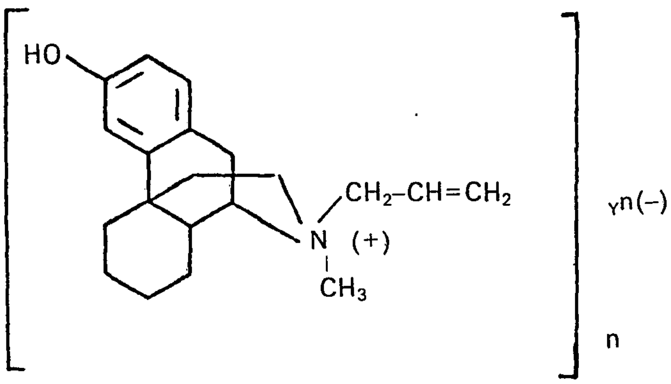

dans laquelle $Y^{n(-)}$ représente l'anion, autre qu'un anion halogène, d'un acide pharmaceutiquement acceptable et n représente le nombre des charges négatives de l'anion, caractérisé en ce qu'on échange l'anion halogène d'un halométhylate de lévallorphane avec l'anion $Y^{n(-)}$ d'un acide pharmaceutiquement acceptable.

6. Procédé selon la revendication 5, caractérisé en ce que l'échange est effectué à l'aide d'une résine échangeuse d'ions.

7. Procédé selon l'une des revendications 5 ou 6, caracterisé en ce qu'on utilise le iodométhylate de lévallorphane comme composé de départ.

8. Composition pharmaceutique à action antagoniste périphérique des opiacés, caractérisé en ce qu'elle contient en tant que principe actif, un composé selon l'une quelconque des revendications 1 à 4.

9. Composition pharmaceutique selon la revendication 8 sous forme d'unité de dosage.

10. Composition pharmaceutique selon la revendication 9, caractérisée en ce qu'elle contient de 1 à 500 mg de principe actif en mélange avec un excipient pharmaceutique.

**Revendications pour l'état contractant AT**

1. Procédé pour la préparation de sels de méthyllévallorphanium de formule:

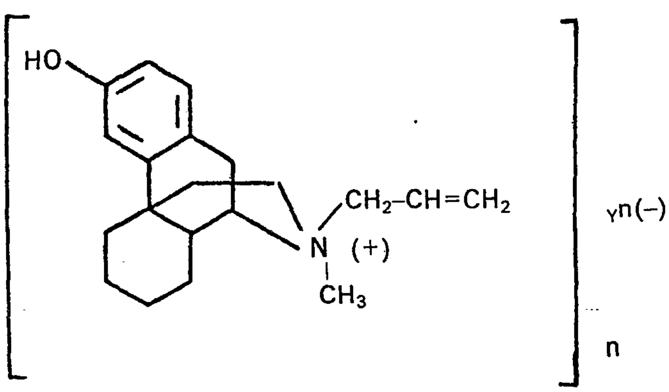

dans laquelle $Y^{n(-)}$ représente l'anion, autre qu'un anion halogène, d'un acide pharmaceutiquement acceptable et n représente le nombre des charges négatives de l'anion; caractérisé en ce qu'on échange l'anion halogène d'un halométhylate de lévallorphane avec l'anion $Y^{n(-)}$ d'un acide pharmaceutiquement acceptable.

2. Procédé selon la revendication 1, caractérisé en ce que l'échange est effectué à l'aide d'une résine échangeuse d'ions.

3. Procédé selon l'une des revendications 1 ou 2, caractérisé en ce qu'on utilise le iodométhylate de lévallorphane comme composé de départ.

4. Procédé selon l'une quelconque des revendications 1 à 3, caractérisé en ce qu'on utilise l'acide méthanesulfonique en tant que donneur d'anion et on isole le méthyllévallorphanium mésylate.

**Patentansprüche für die Vertragsstaaten BE, CH, DE, FR, GB, IT, LI, LU, NL, SE**

1. Methyllevallorphaniumsalz der Formel

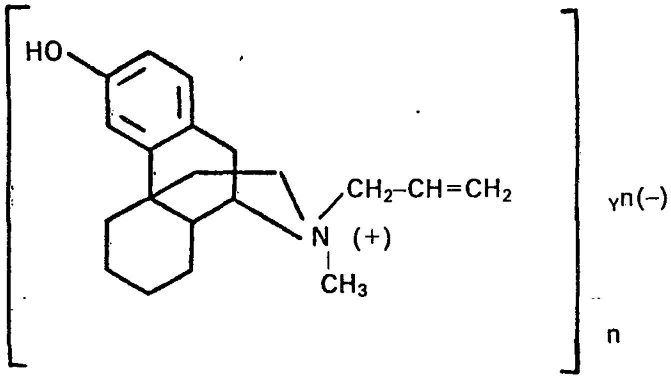

worin $Y^{n(-)}$ das Anion, ausgenommen ein Halogenanion, einer pharmazeutisch akzeptablen Säure darstellt und n die Anzahl der negativen Ladungen des Anions bedeutet.

2. Methyllevallorphanium-mesylat.

3. Saures Methyllevallorphanium-maleat.

4. Methyllevallorphanium-methylsulfat.

5. Verfahren zur Herstellung von Methyllevallorphaniumsalzen der Formel

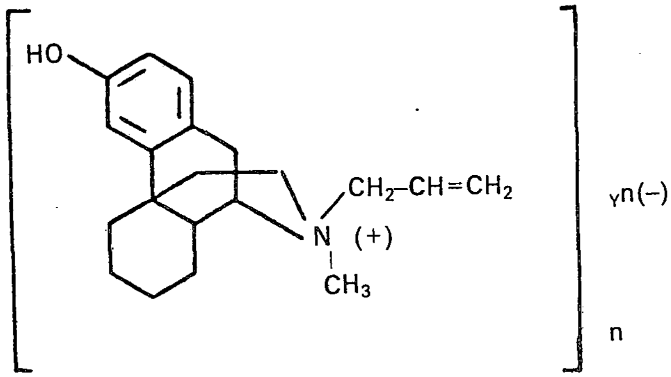

worin $Y^{n(-)}$ das Anion, ausgenommen ein Halogenanion, einer pharmazeutisch akzeptablen Säure darstellt und n die Anzahl der negativen Ladungen des Anions bedeutet, dadurch gekennzeichnet, dass man das Halogenanion eines Halomethylats von Levallorphan mit dem Anion $Y^{n(-)}$ einer pharmazeutisch akzeptablen Säure austauscht.

6. Verfahren nach Anspruch 5, dadurch gekennzeichnet, dass der Austausch mit Hilfe eines Ionenaustauscherharzes erfolgt.

7. Verfahren nach einem der Ansprüche 5 oder 6, dadurch gekennzeichnet, dass man als Ausgangsverbindung Levallorphanjodmethylat verwendet.

8. Pharmazeutische Zusammensetzung mit peripherer antagonistischer Wirkung zu Opiaten, dadurch gekennzeichnet, dass sie als Wirkstoff eine Verbindung nach einem der Ansprüche 1 bis 4 enthält.

9. Pharmazeutische Zusammensetzung nach Anspruch 8 in Dosiseinheitsform.

10. Pharmazeutische Zusammensetzung nach Anspruch 9, dadurch gekennzeichnet, dass sie 1 bis 500 mg Wirkstoff in Mischung mit einem pharmazeutischen Exzipienten enthält.

**Patentansprüche für den Vertragstaat AT**

1. Verfahren zur Herstellung von Methyllevallorphaniumsalzen der Formel

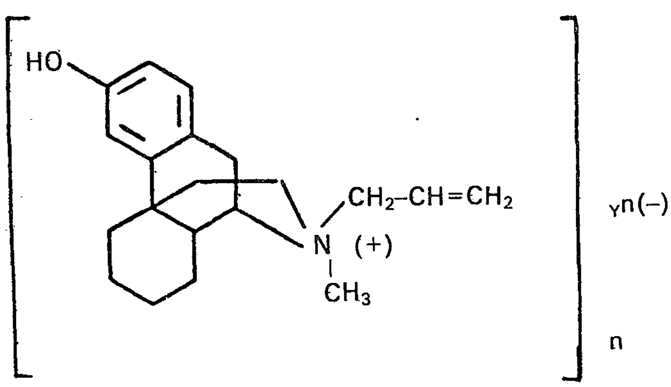

worin $Y^{n(-)}$ das Anion, ausgenommen ein Halogenanion, einer pharmazeutisch akzeptablen Säure darstellt und n die Anzahl der negativen Ladungen des Anions bedeutet, dadurch gekennzeichnet, dass man das Halogenanion eines Halomethylats von Levallorphan mit dem Anion $Y^{n(-)}$ einer pharmazeutisch akzeptablen Säure austauscht.

2. Verfahren nach Anspruch 1, dadurch gekennzeichnet, dass der Austausch mit Hilfe eines Ionenaustauscherharzes erfolgt.

3. Verfahren nach einem der Ansprüche 1 oder 2, dadurch gekennzeichnet, dass man als Ausgangsverbindung Levallorphanjodmethylat verwendet.

4. Verfahren nach einem der Ansprüche 1 bis 3, dadurch gekennzeichnet, dass man als Anionenlieferant Methansulfonsäure verwendet und das Methyllevallorphanium-mesylat isoliert.

**Claims for the contracting states: BE, CH, DE, FR, GB, IT, LI, LU, NL, SE**

1. Methyllevallorphanium salt of formula

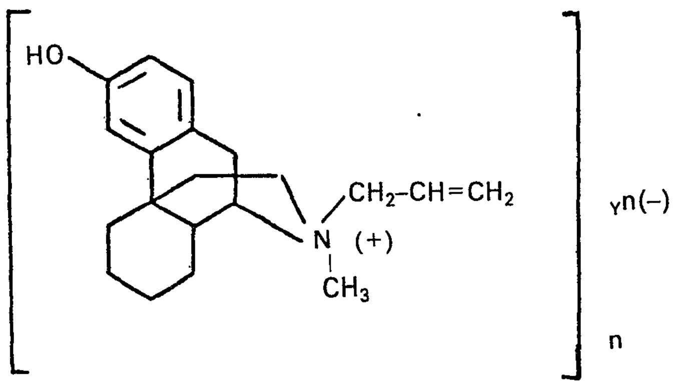

wherein $Y^{n(-)}$ represents the anion, other than halogen anion, of a pharmaceutically acceptable acid and n represents the number of negative charges of the anion.

2. Methyllevallorphanium mesilate.

3. Methyllevallorphanium hydrogen maleate,

4. Methyllevallorphanium methylsulfate.

5. Process for the preparation of methyllevallorphanium salts of formula

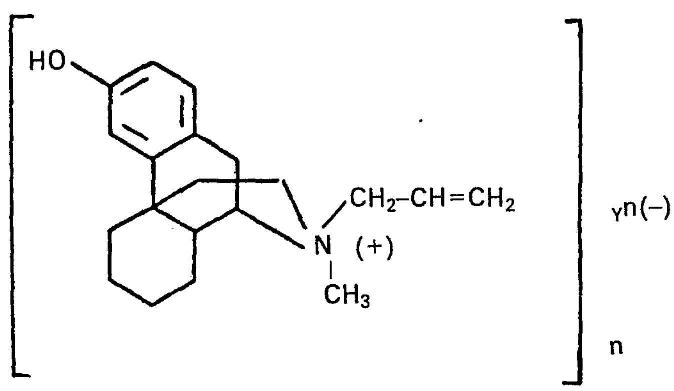

wherein $Y^{n(-)}$ represents the anion, other than halogen anion, of a pharmaceutically acceptable acid and n represents the number of negative charges of the anion, characterized in that it comprises exchanging the halogen anion of a levallorphan halomethylate for the anion $Y^{n(-)}$ of a pharmaceutically acceptable acid.

6. A process according to claim 5, characterized in that the exchange is carried out by means of a ion exchange resin.

7. A process according to claim 5 or 6, characterized in that levallorphan iodomethylate is used as starting compound.

8. Pharmaceutical composition, having an opiate antagonistic peripheral action, characterized in that it contains as active principle a compound according to any one of claims 1 to 4.

9. Pharmaceutical composition according to claim 8, in a dosage unit form.

10. Pharmaceutical composition according to claim 9, characterized in that it contains from 1 to 500 mg of active ingredient in admixture with a pharmaceutical carrier.

**Claims for the contracting state AT**

1. Process for the preparation of methyllevallophanium salts of formula

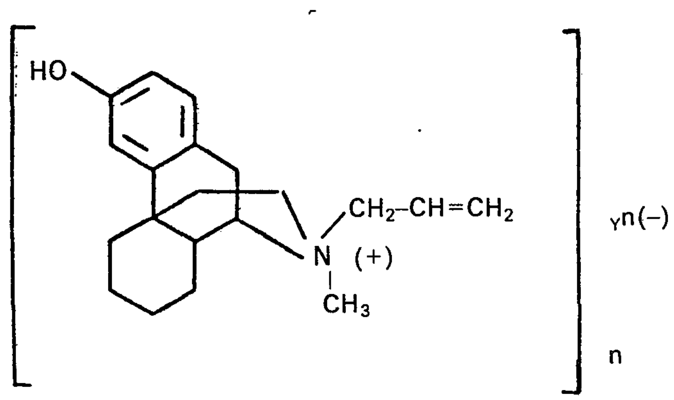

wherein $Y^{n(-)}$ represents the anion, other than halogen anion, of a pharmaceutically acceptable acid and n represents the number of negative charges of the anion; characterized in that it comprises exchanging the halogen anion of a levallorphan halomethylate for the anion $Y^{n(-)}$ of a pharmaceutically acceptable acid.

2. A process according to claim 1, characterized in that the exchange is carried out by means of a ion exchange resin.

3. A process according to any one of claims 1 and 2, characterized in that levallorphan iodomethylate is used as starting compound.

4. A process according to any one of claims 1 to 3, characterized in that the methanesulfonic acid is used as anion donor and the methyllevallorphanium mesilate is isolated.